# EUROPEAN PATENT APPLICATION

(11) **EP 4 653 903 A1**
(43) Date of publication of application: **26.11.2025**
(21) Application number: 24275063.6
(22) Date of filing: 21.05.2024
(51) Int. Cl.: G01R 33/383, G01R 33/38, G01R 33/381

(54) **DEDICATED MAGNETIC RESONANCE DEVICE**

(71) Applicant: Siemens Healthcare Limited, Camberley, Surrey GU15 3YL (GB)
(72) Inventor: Calvert, Simon James, Finstock, Chipping Norton, OX7 3BY (GB); Mallett, Michael, Faringdon, SN7 8EL (GB)
(74) Representative: Siemens Healthineers Patent Attorneys

(57) **Abstract**

The invention relates to a magnetic resonance device (10) comprising a field generation unit (12) configured to generate a main magnetic field including an imaging volume (30), and a support structure (11) configured to structurally support the field generation unit (12), wherein the field generation unit (12) comprises a first magnet (14) and a second magnet (18), the first magnet (14) comprising two magnet segments (14a; 14b) arranged at an angle to each other to form a triangular half-open space (47) which encloses at least a part of the imaging volume (30), and wherein the first magnet (14) and the second magnet (18) are arranged at opposing sides of the imaging volume (18).

## Description

Independent of the grammatical term usage, individuals with male, female or other gender identities are included within the term.

The invention relates to a dedicated magnetic resonance device.

Magnetic resonance tomography represents a prominent imaging method for acquiring images of an interior of an examination object. For carrying out a magnetic resonance measurement, the examination object is usually positioned in a strong and homogeneous static magnetic field (B0 field) of a magnetic resonance device. The static magnetic field may comprise magnetic field strengths of 0.2 Tesla to 7 Tesla, thus aligning nuclear spins inside the examination object along the static magnetic field. For triggering so-called nuclear spin resonances, radiofrequency excitation pulses are emitted into the examination subject. Each radiofrequency excitation pulse causes a magnetization of nuclear spins within the examination object to deviate from the static magnetic field by an amount which is known as the flip angle. A radiofrequency excitation pulse may comprise an alternating (electro-)magnetic field with a frequency which corresponds to the Larmor frequency at the respective static magnetic field strength. Excited nuclear spins may exhibit a rotating and decaying magnetization (nuclear magnetic resonance), which can be detected using dedicated radiofrequency antennas. For spatial encoding of measured data, rapidly switched magnetic gradient fields are superimposed on the static magnetic field.

The received nuclear magnetic resonances are typically digitized and stored as complex values in a k-space matrix. This k-space matrix can be used as a basis for a reconstruction of magnetic resonance images and for determining spectroscopic data. A magnetic resonance image is typically reconstructed by means of a multi-dimensional Fourier transformation of the k-space matrix.

In conventional whole-body scanners, patients are typically accommodated within a bore or between a pair of magnets when a magnetic resonance measurement is to be performed. From a cost and/or space utilization perspective, this may be unsatisfactory, especially if the examination is restricted to a body region of the patient which is significantly smaller than an imaging volume provided by the whole-body scanner. Furthermore, patients with a claustrophobic condition and/or children may not tolerate being positioned in a confined imaging space for a prolonged period of time.

It is therefore an object of the invention to provide a magnetic resonance device with enhanced openness and/or accessibility for imaging of dedicated body regions of a patient.

This object is achieved by a magnetic resonance device according to the invention. Further advantageous embodiments are specified in the dependent claims.

The inventive magnetic resonance device comprises a field generation unit configured to generate a main magnetic field including an imaging volume, and a support structure configured to structurally support the field generation unit. The field generation unit comprises a first magnet and a second magnet. The first magnet comprises two magnet segments arranged at an angle to each other to form a triangular half-open space which encloses at least a part of the imaging volume.

The first magnet may comprise or consist of a permanent magnet or an array of permanent magnets.

A permanent magnet may consist of a magnetic material such as AlNiCo (aluminium-nickel-cobalt), NdFeB (neodymium-iron-boron), SmCo (samarium-cobalt), or the like. A magnet segment of the first magnet may comprise the shape of a bar, a cuboid, a cylinder, a prism, or the like. Bar-shaped permanent magnets may favourably provide a low-cost solution for generating a magnetic field. According to an embodiment, the first magnet is composed of smaller, stacked permanent magnets or an array of permanent magnets. An array of permanent magnets may represent a Halbach array. The use of a permanent magnet may favourably avoid costs and space required for cooling equipment usually associated with superconducting magnets and electromagnets.

According to an embodiment, the first magnet comprises or consists of an electromagnet.

An electromagnet may represent a non-superconducting magnet. Particularly, an electromagnet may comprise an electrical conductor wound around a magnetic core made of, for example, a ferromagnetic or ferrimagnetic material. The magnetic core of the electromagnet may comprise a cylindrical shape, a cuboid shape, a prism shape, or any other suitable shape. In providing an electromagnet, a magnetic field strength of a magnetic field provided by the first magnet may be increased in comparison to a permanent magnet of comparable size. A higher magnetic field strength may favourably allow for an acquisition of magnetic resonance images with an improved image quality and/or signal-to-noise ratio.

According to a further embodiment, the first magnet comprises or consists of a superconducting magnet.

For example, the first magnet may comprise or consist of a high temperature superconducting material and/or a low temperature superconducting material. A superconducting magnet may comprise one or more coils of superconducting wire. The superconducting wire may be thermally coupled to a cryocooler configured to maintain a temperature of the superconducting wire below a predefined value. The one or more coils of superconducting wire may be arranged in a variety of shapes, such as a solenoid, a substantially planar loop, or a tubular segment. In using a superconducting magnet, the magnetic field strength of a magnetic field provided by the first magnet can favourably be increased in comparison to a permanent magnet or an electromagnet of comparable size.

The second magnet may comprise or consist of a permanent magnet, an electromagnet, or a superconducting magnet according to an embodiment described above. In a preferred embodiment, the second magnet consists of a permanent magnet.

The two magnet segments of the first magnet are arranged in such a way to form a triangular half-open space. The triangular half-open space may enclose at least a part of the imaging volume.

According to an embodiment, the triangular half-open space may be confined by the first magnet in at least one spatial direction, preferably at least two spatial directions. For example, a first magnet segment of the first magnet may confine the triangular half-open space in a first spatial direction, and a second magnet segment of the first magnet may confine the triangular half-open space in a second spatial direction. It is conceivable that the second magnet confines the triangular half-open space in a third spatial direction. Preferably, the first spatial direction, the second spatial direction and the third spatial direction differ from each other.

In a preferred embodiment, the triangular half-open space formed between the at least two magnet segments of the first magnet provides an opening for a patient to access the imaging volume.

The angle between the two magnet segments may comprise a value between 10 degrees and 180 degrees, preferably between 60 and 120 degrees. Particularly, the two magnet segments may be arranged in such a way to form an open triangle or a 'V'. In providing a field generation with a triangular or `V'-shape, an accessibility of the imaging volume may favourably be improved in comparison to conventional magnetic resonance devices with minimal impact on an efficiency of the field generation unit.

The support structure may be configured to maintain a relative position between the two magnet segments of the first magnet. The support structure may also be configured to maintain a relative position between the first magnet and the second magnet. Particularly, the support structure may be attached to a first magnet segment and a second magnet segment of the first magnet via a form-locking connection, a force-locking connection and/or a material bond. The support structure may be mechanically connected to the second magnet in a similar fashion. In a preferred embodiment, the support structure is screwed, bolted, clamped and/or glued to the first magnet and the second magnet.

The support structure may comprise or consist of any material capable of withstanding the gravitational, electromagnetic and/or mechanical forces exerted on or by the first magnet and/or the second magnet of the field generation unit. Typical examples of suitable materials are metals, such as iron, steel, or stainless steel, but also metal alloys, ceramics, as well as synthetic materials or composite materials.

According to an embodiment, the support structure comprises a yoke. The yoke may enhance, modify and/or restrict a magnetic flux density in a specific region of the magnetic resonance device, such as the triangular half-open space and/or the imaging volume. It is conceivable, that the yoke is made of a material with high magnetic permeability. Particularly, the yoke may comprise a high amount of iron and/or other ferromagnetic materials. Examples of materials with high magnetic permeability are metals, such as iron, cobalt, or nickel, but also alloys of these metals. The support structure and/or yoke may be designed to enhance, restrict, or modify the magnetic field generated by the field generation unit.

According to a further embodiment, the support structure comprises two end plates attached the two magnet segments of the first magnet. For example, a first end plate may be attached to a first magnet segment and a second end plate may be attached to a second magnet segment of the first magnet. The two end plates may be formed as separate pieces or separate elements attached to the support structure. However, the two end plates and the support structure may form a coherent or monolithic structure. In one example, the first end plate is attached to the second end plate via a form-locking mechanical connection, a force-locking mechanical connection and/or a material bond.

The support structure may be configured to mount the field generation unit and/or the magnetic resonance device to a part of an examination room. For example, the support structure may be configured to mount the first magnet and/or the second magnet to a floor, a wall and/or a ceiling of the examination room.

An imaging volume may be characterized by a predefined magnetic field direction and/or a predefined magnetic field strength. For example, the imaging volume may comprise a volume having a substantially uniform magnetic field direction and/or a substantially uniform magnetic field strength. The imaging volume may correspond to a homogenous volume within the magnetic field generated via the field generation unit. Particularly, the imaging volume may represent an isocentre of the magnetic resonance device. It is also conceivable, that the imaging volume comprises a predefined or static magnetic gradient field. Such a magnetic gradient field may be used for spatial encoding of magnetic resonance signals acquired from an examination object positioned within the imaging volume.

According to the invention, the first magnet and the second magnet are arranged at opposing sides of the imaging volume.

For example, the first magnet and the second magnet may be arranged in such a way to confine the imaging volume from at least two sides and/or from at least two opposing spatial directions. The imaging volume may be at least partially arranged within the triangular half-open space formed by the two magnet segments of the first magnet. Preferably, the second magnet is arranged outside the triangular half-open space formed by the two magnet segments of the first magnet.

According to an embodiment, the field generation unit is configured to provide a small, targeted imaging volume specifically adapted for imaging of a specific body region of a patient. The imaging volume of the magnetic resonance device may be substantially smaller than an imaging volume of a conventional magnetic resonance device. For example, a maximum diameter of a sphere with the same volume as the imaging volume (diameter of spherical volume - DSV) may be smaller than 25 cm, smaller than 20 cm, smaller than 15 cm, smaller than 10 cm, smaller than 8 cm, smaller than 6 cm, or smaller than 5 cm. A minimum diameter of a sphere with the same volume as the imaging volume may exceed 2 cm, or 5 cm, or 8 cm, or 10 cm.

The imaging volume may comprise a spherical or non-spherical shape, e. g. an ellipsoidal shape, a conical shape, a toroidal shape, a cuboid shape, a star shape, or any shape obtained by twisting and/or deforming of one of the mentioned shapes. In providing a field generation unit configured to provide a small, targeted imaging volume, an overall size of the magnetic resonance device may favourably be reduced in comparison to a conventional magnetic resonance device.

In a preferred embodiment, the inventive magnetic resonance device is configured to acquire magnetic resonance imaging data from an examination object, particularly a patient, positioned within the triangular half-open space. For this purpose, the magnetic resonance device may comprise further components usually required for performing a magnetic resonance examination and for processing acquired magnetic resonance imaging data. In particular, the magnetic resonance device may comprise a control unit configured to control the magnetic resonance device to carry out a magnetic resonance measurement and acquire magnetic resonance signals from the examination object. The magnetic resonance device may also comprise a processing unit configured to reconstruct a magnetic resonance image based on the acquired magnetic resonance signals. The triangular half-open space may correspond to an image acquisition region of the magnetic resonance device.

The inventive magnetic resonance device may represent a dedicated scanner configured to perform a magnetic resonance imaging examination of a specific body region or a plurality of specific body regions of a patient. For example, a specific body region may comprise a heart, an eye, a tooth, multiple teeth, a jaw, a prostate, and the like.

In providing an inventive magnetic resonance device (i. e. an inventive dedicated scanner) configured for imaging one or more specific body regions of a patient, an overall dimension of the magnetic resonance device may favourably be reduced in comparison to conventional, whole-body magnetic resonance devices. Furthermore, the inventive magnetic resonance device may be less expensive and/or easier to install in confined spaces in comparison to conventional magnetic resonance devices.

A field generation unit comprising a first magnet and a second magnet arranged at opposite sides of the imaging volume may favourably allow for the imaging volume to be moved away from a corner of the triangular half-open space formed by the first magnet. Thus, an accessibility of the imaging volume may favourably be improved in comparison to a field generation unit consisting of just the first magnet.

Particularly, the inventive magnetic resonance device may favourably allow for anatomic regions of a patient to be examined, which would not fit into the corner of the triangular half-open space. Furthermore, the inventive magnetic resonance device may favourably allow imaging of organ structures located deeper within a body of the patient, such as a heart and/or a prostate. Such organ structures may not be able to be examined in a dedicated scanner comprising just a 'V'-shaped magnet due to their position inside the body and a predetermined distance between the imaging volume and the corner of the triangular half-open space.

According to an embodiment of the magnetic resonance device, a direction of a horizontal polarization of the second magnet is opposite to a horizontal polarization of the first magnet.

The second magnet may represent a single-sided magnet or a directional magnet.

The term single-sided magnets may refer to a magnet having a higher magnetic field strength on a first side and a weaker magnetic field strength or substantially no magnetic field on a second side opposite to the first side. For example, the second side of the single-sided magnet may be covered with an iron sheet providing a shielding effect and/or deflecting the magnetic field in a direction towards the first side. Thus, the first side may exhibit a significantly increased magnetic field strength in comparison to the second side.

A directional magnet may comprise a first pole and a second pole, the first pole having a higher magnetic field strength in comparison to the second pole.

A magnetic field generated via the second magnet may be located predominantly or entirely on a side of the second magnet facing towards the imaging volume.

Preferably, a main direction of magnetic field lines of the magnetic field provided via the first magnet within the triangular half-open space corresponds to a main direction of magnetic field lines of the magnetic field provided by the second magnet on the side of the second magnet facing towards the imaging volume.

An inventive field generation unit comprising a first magnet and a second magnet having opposite horizontal polarizations may favourably allow for the homogenous volume of the magnetic resonance device to be increased and/or a spatial location of the homogenous volume to be adjusted. Thus, the magnetic resonance device may allow for imaging of larger objects or larger portions of a body of patient.

According to a further embodiment of the inventive magnetic field generation unit, the support structure is configured to variably change a spatial position of the second magnet relative to the first magnet.

For example, the support structure may comprise a first section attached to the first magnet, and a second section attached to the second magnet. The first section may be mechanically connected to the second section. However, the first section and the second section may be mechanically disjoint.

According to an embodiment, the first section is mechanically separated from the second section. In a further embodiment, the first section and the second section are mechanically coupled via the support structure.

The first section may represent a portion of the support structure configured to maintain the angle between the two magnet segments of the first magnet. For example, the first section may comprise one or more end plates according to an embodiment described above. Preferably, the first section is configured to provide mechanical support to the first magnet and/or carry the first magnet. The first section may be mechanically connected or attached to the first magnet.

The second section may be configured to provide mechanical support to the second magnet and/or carry the second magnet. Particularly, the second section may be mechanically connected or attached to the second magnet.

According to an embodiment, the first section and the second section are mechanically connected. For example, the first section may be directly attached to the second section. In attaching the first section to the second section, a risk of movement of the second section relative to the first section may be reduced. Thus, a degradation of a magnetic homogeneity within the imaging volume due to relative motion between the first magnet and the second magnet may favourably be avoided.

However, the first section may also be mechanically coupled to the second section. For example, the first section may be attached to a third section of the support structure attached to the second section. In other words, the first section may be spaced from the second section via the third section. The third section may comprise a static support element, such as a bar, a beam, a strut, or any other suitable support element. However, the third section may also comprise a dynamic support element, particularly an adjusting unit according to an embodiment described below. In allowing the second magnet to be moved relative to the first magnet, an access to the imaging volume may favourably be facilitated.

According to a preferred embodiment of the inventive magnetic resonance device, the first section is mechanically coupled to the second section, and the support structure comprises an adjustment unit configured to variably change a relative position between the first section and the second section.

The adjustment unit or adjusting mechanism may be configured to variably change the spatial position of the second magnet relative to the first magnet. For example, the adjustment unit may comprise a moveable joint, such as a hinge, a pivot, or a swivel. The moveable joint may be configured to enable the second magnet to be guided along a predefined motion trajectory relative to the first magnet. For example, the predefined motion trajectory may correspond to a section of an elliptical arc or a section of a circular arc.

According to an embodiment, the adjustment unit comprises a first portion attached to the first section of the support structure, and a second portion attached to the second section of the support structure. The adjustment unit may comprise any suitable form of moveable joint configured to allow for a relative movement of the second magnet relative to the first magnet.

A moveable joint, particularly a hinge or swivel, may represent a particularly cost-effective embodiment of an adjustment unit. Furthermore, a hinge or swivel may favourably provide a restriction of the movement of the second magnet to a predefined motion trajectory. Thus, an undesired misalignment between the first magnet and the second magnet may favourably be avoided.

According to an embodiment of the inventive magnetic resonance device, the adjustment unit comprises a guide mechanism.

The guide mechanism may comprise a rail or a slide mechanism. Particularly, the guide mechanism may comprise a guide element defining a substantially linear motion trajectory of the second magnet. For example, the guide element may comprise or consist of one or more rods, bars, rails, bearings, or the like.

According to an embodiment, the second section of the support structure may comprise or form a slide or skid configured to slide along the guide element. The guide mechanism and/or the guide element may correspond to a third section according to an embodiment described herein.

A guide mechanism may favourably enable the second magnet to be variably moved relative to the first magnet. Thus, an access of a patient to the triangular half-open space may be facilitated. Furthermore, a guide mechanism may favourably provide an improved mechanical support to the second magnet along the motion trajectory defined by the guide element.

It is conceivable that the adjustment unit comprises a drive or motor configured to variably move the second magnet relative to the first magnet. In certain embodiments, the adjustment unit may be mechanically connected or mechanically coupled to the first magnet and the first section of the support structure. However, the adjustment unit may also be mechanically separated from the first magnet and the first section of the support structure. For example, the adjustment unit may be carried by a second section of the support structure mechanically separated from the first section and configured to carry the second magnet and/or mount the second magnet to a floor, a wall and/or a ceiling of an examination room.

In a preferred embodiment, the adjustment unit is configured to automatically adjust the spatial position of the second magnet relative to the first magnet in dependence of a control signal. For example, the adjustment unit may comprise a motor or drive configured to receive a control signal provided via a control unit of the inventive magnetic resonance device.

In providing an adjustment unit configured to automatically adjust a spatial position of the second magnet relative to the first magnet, the first magnet and the second magnet may favourably be arranged in one or more desired spatial arrangements in a time-efficient and/or reproduceable manner.

According to an embodiment, the inventive magnetic resonance device comprises a stop element configured to restrict or limit a movement of the second magnet along a motion trajectory defined by the adjustment unit.

The stop element may be configured to prevent movement of the second magnet beyond a predefined spatial position along the motion trajectory. For example, the stop element may be configured to prevent movement of the second magnet past a measurement position and/or past an opening position according to an embodiment described below.

The stop element may comprise a suitable mechanism configured to limit the movement of the second magnet along the motion trajectory. For example, the stop element may comprise a locking element, such as a bolt, a block, a plate, or an anchor. The stop element may also comprise a retaining element, a damping element, or the like. It is conceivable that the stop element is mechanically connected the support structure and/or the adjustment unit, particularly the guide element.

A stop element may favourably allow for the second magnet to be moved between predetermined spatial positions defined by the stop element. Thus, the second magnet may favourably be moved to a desired spatial position in a time-efficient manner. Furthermore, the stop element may favourably prevent the second magnet from being arranged in an undesired spatial position and/or from injuring a patient accommodated within the triangular half-open space.

According to a preferred embodiment of the inventive magnetic resonance device, the support structure is configured to arrange the second magnet in an opening position and a measurement position.

Preferably, the support structure comprises an adjusting unit configured to arrange the second magnet in the opening position and the measurement position. The adjusting unit may be implemented according to an embodiment described above.

The opening position may be characterized by a relative spatial arrangement of the second magnet to the first magnet that allows a patient to access the triangular half-open space. Particularly, the opening position may characterize a spatial arrangement of the field generation unit which avoids an obstruction of an opening to the triangular half-open space by the second magnet. For example, the second magnet may be arranged at a predefined distance to the opening of the triangular half-open space. It is also conceivable that the second magnet is arranged off-axis and/or at an angle with respect to the first magnet when arranged in the opening position.

The measurement position may be characterized by a relative spatial arrangement of the second magnet to the first magnet, in which the magnetic field provided by the first magnet and the magnetic field provided by the second magnet overlap at least partially within the triangular half-open space. It is also conceivable that the measurement position characterizes a spatial position of the second magnet relative to the first magnet which allows the magnetic fields of the first magnet and the second magnet to interact and/or form a common or shared homogenous volume or imaging volume at least partially arranged within the triangular half-open space. Particularly, the measurement position may be characterized by the second magnet being arranged about the opening of the triangular half-open space. For example, the second magnet may block or obstruct the opening of the triangular half-open space when arranged in the measurement position. The measurement position may also be characterized in that a projection of a cross-sectional area of the opening of the triangular half-open space along a symmetry plane of the first magnet, particularly a symmetry plane dividing the two magnet segments (e. g. a symmetry plane corresponding to a Y-direction of the magnetic resonance device), has a non-empty intersection with the second magnet.

In providing a support structure configured to arrange the second magnet in an opening position, an accessibility of the imaging volume may favourably be improved. A second magnet arranged in a measurement position may favourably allow for a provision of higher magnetic fields strengths and/or an increased homogenous volume in comparison to magnetic resonance devices comprising single-sided magnets or a field generation unit consisting of just the first magnet.

According to an embodiment of the inventive magnetic resonance device, the field generation unit is configured in such a way to cause a spatial position of the imaging volume to change when the spatial position of the second magnet is changed relative to the first magnet.

Moving the second magnet from the opening position to the measurement position may cause a shape, a size and/or a spatial position of the imaging volume within the triangular half-open space to change. Particularly, moving the second magnet between the opening position and the measurement position may cause a spatial position of a geometric centre of the imaging volume to be changed.

By variably moving the second magnet relative to the first magnet, an interaction and/or overlap between the magnetic fields generated by the first magnet and the second magnet may be changed. Thus, the imaging volume may comprise different shapes, different sizes and/or different spatial positions in dependence of the spatial position of the second magnet relative to the first magnet.

In providing a predetermined motion trajectory of the second magnet relative to the first magnet, the properties of the imaging volume provided via the field generation unit may be changed to accommodate for differently sized patients and/or different body regions.

The inventive magnetic field generation unit may favourably allow for adjusting a shape, a size and/or a spatial position of a homogenous volume in dependence of an object, particularly a body region of a patient, to be examined.

In a preferred embodiment of the inventive magnetic resonance device, the field generation unit is configured in such a way to cause the imaging volume to move away from a corner of the triangular half-open space when the second magnet is moved from the opening position to the measurement position.

The field generation unit may be configured in such a way to cause the imaging volume to move towards the opening of the triangular half-open space when the second magnet is moved from the opening position to the measurement position.

Preferably, the field generation unit is configured in such a way to cause the imaging volume to at least partially move out of the triangular half-open space towards the opening of the triangular half-open space when the second magnet is transferred from the opening position to the measurement position.

The inventive magnetic resonance device may favourably allow for imaging of objects or body parts of patients whose external dimensions exceed an available space close to the corner of the triangular half-open space. Furthermore, in moving the imaging volume away from the corner of the first magnet, an access to the imaging volume may be improved. Thus, carrying out an imaging examination of patients with disabilities or obesity may favourably be facilitated.

According to an embodiment of the inventive magnetic resonance device, the support structure is configured to arrange the second magnet in a first measurement position and a second measurement position. A spatial location of the second magnet in the first measurement position differs from a spatial position of the second magnet in the second measurement position.

The support structure may be configured to arrange the second magnet in a plurality of measurement positions. In each measurement position of the plurality of measurement positions, the second magnet may be arranged in a different spatial position relative to the first magnet.

It is conceivable that a shape, a size and/or a spatial position of the imaging volume provided by the field generation unit is different in each measurement position. Particularly, each measurement position may provide an imaging volume with a predefined shape and/or a predefined spatial position relative to a volume defined by the triangular half-open space.

The inventive magnetic resonance device may favourably allow for a selection of a measurement position in dependence of a body region of a patient to be imagined. Thus, a positioning of a patient for a magnetic resonance measurement may be facilitated and/or a number of body regions that can be examined via the inventive magnetic resonance device may favourably be increased.

According to an embodiment, the inventive magnetic resonance device comprises a radiofrequency system including at least one radiofrequency antenna configured to transmit and/or receive radiofrequency radiation.

The radiofrequency system may comprise one or more radiofrequency antennas configured to transmit radiofrequency excitation pulses and/or receive magnetic resonance signals. Preferably, the radiofrequency system comprises at least two radiofrequency antennas. The at least two radiofrequency antennas may be arranged at two opposing sides of the field generation unit, particularly at two separate sections of the first magnet. For example, a first radiofrequency antenna may be attached to a first magnet segment of the first magnet, and a second radiofrequency antenna may be attached to a second magnet segment of the first magnet. It is also conceivable, that at least one radiofrequency antenna is carried by and/or attached to the support structure and/or the second magnet.

According to an embodiment, the radiofrequency system is designed to match a contour and/or a surface of an object to be imaged. Thus, a distance between the at least one radiofrequency antenna and the surface of the imaging object may favourably be decreased. However, the at least one radiofrequency antenna may also be shaped to match a surface directed towards the imaging volume of the first magnet and/or the second magnet.

According to a further embodiment, the inventive magnetic resonance device comprises a gradient field system including at least one gradient coil configured to generate at least one magnetic gradient field.

The gradient field system may comprise one or more gradient coils configured to generate a magnetic gradient field along an x-direction, a y-direction and/or a z-direction. In one embodiment, the at least one gradient coil may comprise a coil of wire configured to generate a magnetic field when a current is applied. It is conceivable, that the gradient field system is carried by the supporting structure, the first magnet and/or the second magnet. For example, the at least one gradient coil may form a layer on the first magnet segment and/or the second magnet segment of the first magnet. Particularly, the at least one gradient coil may be at least partially recessed into a surface or pole face of the first magnet, e. g. a surface or pole face of the first magnet segment and/or the second magnet segment. A conceivable value for the maximum gradient field strength provided via the gradient field system may range between 10 and 30 mT/m and a slew rate may range between 10 and 30 T/m/s. However, this range can also be upwardly or downwardly transgressed on a case-by-case basis.

Further advantages and details of the present invention may be recognized from the embodiments described below as well as the drawings. The figures show:
- Fig. 1: a schematic representation of an embodiment of an inventive magnetic resonance device,
- Fig. 2: a schematic representation of an embodiment of an inventive magnetic resonance device,
- Fig. 3: a schematic representation of an embodiment of an inventive magnetic resonance device,
- Fig. 4: a schematic representation of an embodiment of an inventive magnetic resonance device,
- Fig. 5: a schematic representation of an embodiment of an inventive magnetic resonance device,
- Fig. 6: a schematic representation of an embodiment of an inventive magnetic resonance device,
- Fig. 7: a schematic representation of an embodiment of an inventive magnetic resonance device,
- Fig. 8: a schematic representation of an embodiment of an inventive magnetic resonance device.

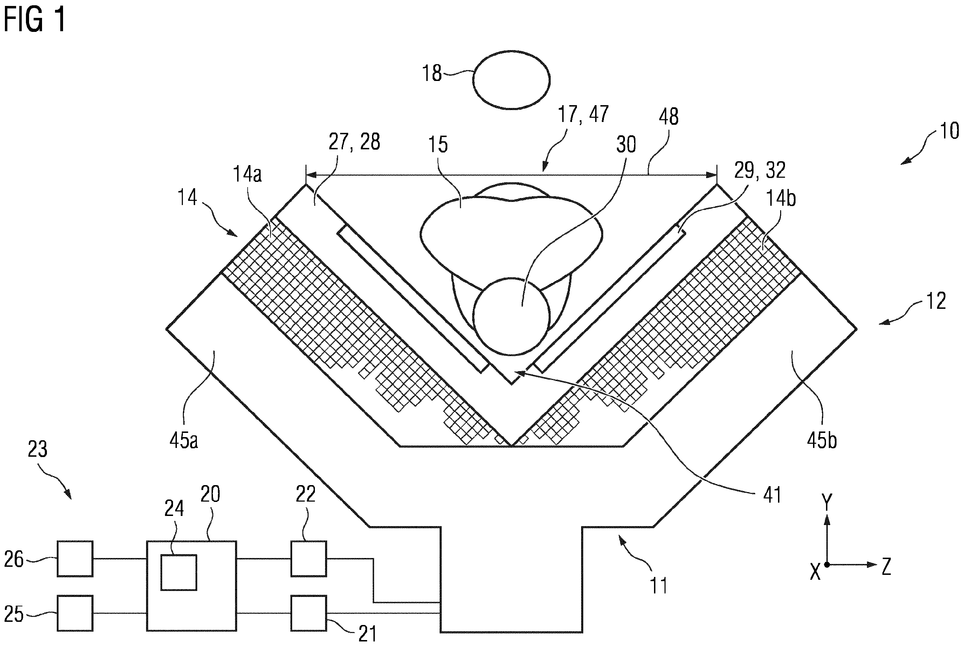

Fig. 1 depicts a schematic representation of an inventive magnetic resonance device 10 configured to perform a magnetic resonance imaging examination of a head region, particularly a jaw region or an eye region, of a patient 15. However, an application of the magnetic resonance device 10 for imaging of a head region of a patient 15 is to be understood as an example. The inventive magnetic resonance device 10 may also be configured to perform
- cardiac imaging of a heart,
- mammography imaging of a breast,
- neurological imaging of a brain and/or a spine,
- orthopedic imaging of joints (e. g. knee, shoulder, elbow),
- ophthalmologic imaging of an eye, an optic nerve and/or related anatomy,
- dental imaging of a jaw and/or one or more teeth (preferably supported via a dedicated intraoral or extraoral radiofrequency antenna),
- urologic imaging of a prostate (e. g. by orienting the field generation unit 12 in such a way that a patient 15 can be accommodated in the triangular half-open space 47 in a sitting position),
- or imaging of other body regions of the patient 15 (e. g. by using a dedicated radiofrequency antenna 32).

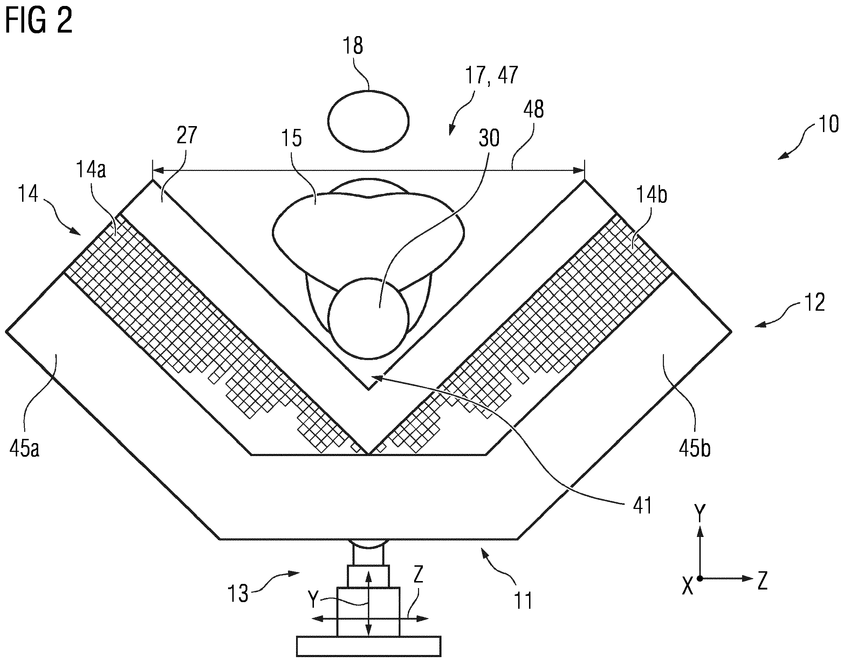

The field generation unit 12 of the magnetic resonance device 10 may comprise a positioning unit 13 configured to position and/or orient the field generation unit 12 in dependence of a diagnostically relevant body region of the patient 15 (see Fig. 2). The positioning unit 13 may carry and/or be attached to the support structure 11.

The inventive magnetic resonance device 10 may be used in veterinary care to visualize body regions of animals, for example extremities of large animals like horses or cows. The opening 48 of the triangular half-open space 47 comprising the imaging volume 30 may be oriented in such a way to accommodate the animal and/or facilitate imaging of the animal (e. g. a cat, a dog, a hamster, etc.).

The inventive magnetic resonance device 10 depicted in Fig. 1 comprises a field generation unit 12. The field generation unit 12 comprises a magnet 14 having a first magnet segment 14a and a second magnet segment 14b, and a magnet 18.

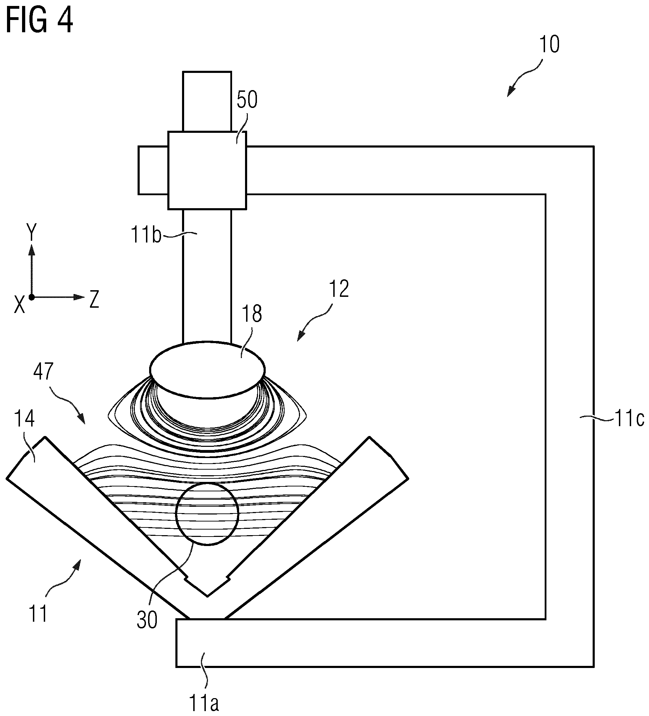

The first magnet segment 14a and the second magnet segment 14b are carried by a support structure 11, which is configured to maintain a predefined spatial arrangement of the first magnet segment 14a and the second magnet segment 14b. The support structure 11 may comprise or consist of an iron yoke. In certain embodiments, the support structure 11, the magnet 14 and the magnet 18 form a coherent structure. However, the support structure 11 may also comprise an adjusting unit 50 configured to move the magnet 18 relative to the magnet 14 (see Fig. 4 to 7).

In the embodiment depicted in Fig. 1, the support structure 11 comprises two end plates 45a and 45b, which are arranged at an angle of more than 0 degrees and less than 180 degrees. The angle between the end plates 45a and 45b may be in the range of 30-150 degrees, preferably in the range of 40-60 degrees, 60-80 degrees, 80-100 degrees, or 100-120 degrees. Preferably, the angle between the end plates 45a and 45b corresponds to the angle between the first magnet segment 14a and the second magnet segment 14b. In one embodiment, the angle between the first magnet segment 14a and the second magnet segment 14b is essentially 90 degrees. However, the angle between the first magnet segment 14a and the second magnet segment 14b may also be less than 90 degrees or exceed 90 degrees. In the cross-sectional view depicted in Fig. 1, the field generation unit 12 comprises the shape of an 'L' or a `V'. However, the field generation unit 12 may also comprise a rounded corner 41 providing a 'U'-shape in the cross-sectional view.

A width of the opening 48 of the triangular half-open space 47 may exceed 60 cm, 80 cm, 100 cm, 120 cm, or 140 cm, thus providing enough clearance to facilitate an access of a patient 15 to the imaging volume 30.

In the example depicted in Fig. 1, the triangular half-open space 47 comprises a corner 41 or nook where the end plates 45a and 45b are connected and/or attached to one another. In cases where the field generation unit 12, particularly the magnet 18, is arranged in an opening position as shown in Fig. 1, the imaging volume 30 may be located in proximity to the corner 41 or nook of the triangular half-open space 47. In arranging the field generation unit 12 and/or the magnet 18 in the measurement position (see Figs. 4 to 6), the imaging volume 30 may be moved away from the corner 41 towards the opening 48 of the triangular half-open space.

A free volume between the first magnet segment 14a and the second magnet segment 14b may represent an image acquisition region 17 configured to receive the examination object 15, e. g. a body region of a patient 15. In the example depicted in Fig. 1, the imaging volume 30 is positioned within the image acquisition region 17 and confined by the first magnet 14 in two spatial directions. The second magnet 18 may confine the imaging volume 30 in a third spatial direction. The image acquisition region 17 may correspond to the triangular half-open space 47 formed by the first magnet segment 14a and the second magnet segment 14b.

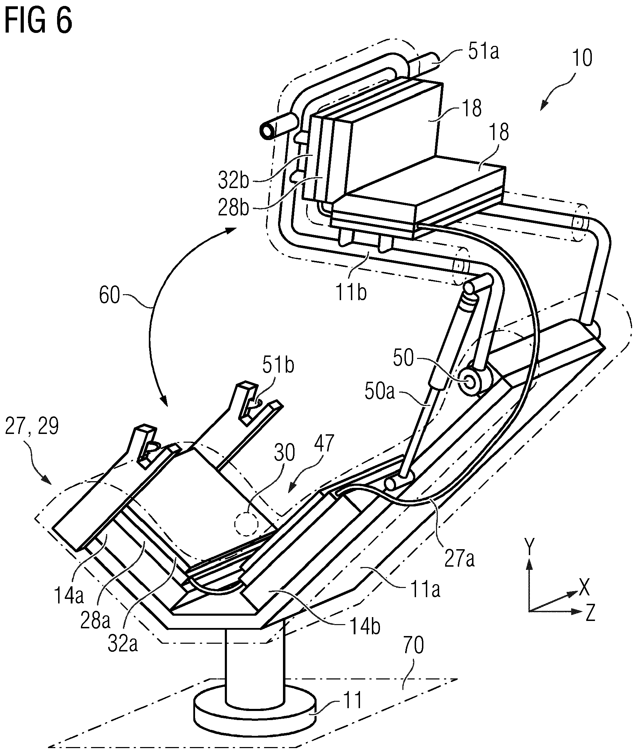

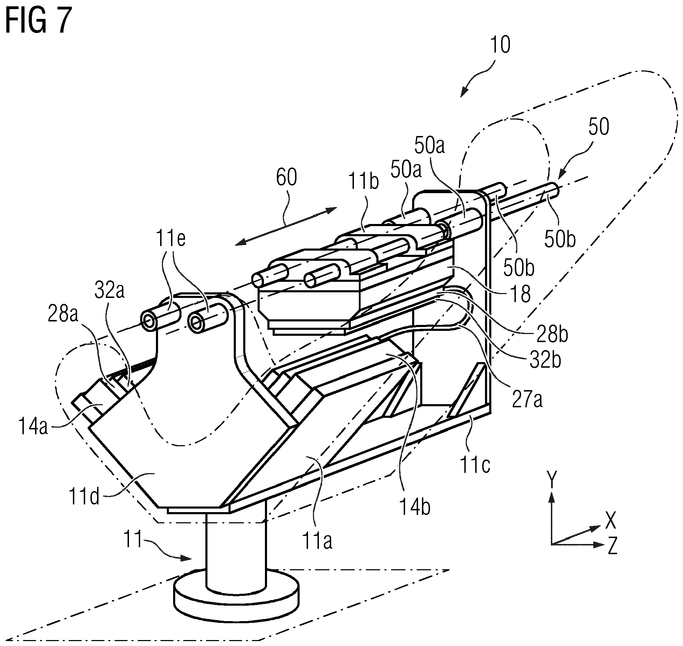

The field generation unit 12 is configured to generate a magnetic field in the image acquisition region 17. In the example shown in Fig. 1, the field generation unit 12 comprises a gradient field system 27 including at least one gradient coil 28 (see Figs. 6 and 7) configured to generate a magnetic gradient field within the image acquisition region 17. The magnetic gradient field may be used for spatial encoding of magnetic resonance signals acquired during a magnetic resonance imaging examination.

In a preferred embodiment, the field generation unit 12 further comprises a radiofrequency system 29 including at least one radiofrequency antenna 32 (see Figs. 6 and 7) configured to emit a radiofrequency excitation pulse into the image acquisition region 17. The at least one radiofrequency antenna 32 may be configured to receive magnetic resonance signals from the image acquisition region 17, particularly the imaging volume 30. According to an embodiment, the at least one radiofrequency antenna 32 is implemented as a local coil (not shown) configured to be arranged in proximity to a diagnostically relevant body region of the patient 15.

In the embodiment depicted in Fig. 1, the magnetic resonance device 10 comprises a control unit 20 configured to control the field generation unit 12, the gradient field system 27, and the radiofrequency system 29. For example, the control unit 20 may be configured to control a gradient control unit 21 electrically connected to the gradient field system 27 and a radiofrequency control unit 22 electrically connected to the radiofrequency system 29. It is also conceivable, that the gradient control unit 21 and the radiofrequency control unit 22 are integrated within the control unit 20. In a preferred embodiment, the control unit 20 is configured to control the magnetic resonance device 10 to perform an imaging examination of a patient 15 arranged within the image acquisition region 17.

The control unit 20 may comprise a processing unit 24 configured to coordinate an acquisition of magnetic resonance data and/or a reconstruction of magnetic resonance images based on magnetic resonance data acquired from the imaging volume 30. It is conceivable, that the processing unit 24 is configured to evaluate and process data, such as acquired magnetic resonance signals or magnetic resonance data, but also imaging parameters and/or imaging protocols of imaging sequences. The control unit 20 may comprise a controller, a microcontroller, an analog circuit, a logic unit, and the like. The processing unit 24 may comprise a processor, such as a CPU, a GPU and the like. It is also conceivable, that the control unit 20 and/or the processing unit 24 comprise a memory and/or an internal storage, such as a RAM, a ROM, a PROM, an EPROM, an EEPROM, a flash memory, as well as an HDD, an SSD, or the like.

The magnet resonance device 10 may comprise an output unit 25 configured to output or display control information, such as imaging parameters and/or magnetic resonance data. Preferably, the output unit 25 comprises at least one monitor configured to display the control information, but also magnetic resonance data and/or magnetic resonance images acquired via the magnetic resonance device 10, to an operator of the magnetic resonance device 10. The magnetic resonance device 10 may further comprise an input unit 26 configured to receive information and/or parameters entered by the operator during an imaging examination.

It is conceivable, that the magnetic resonance device 10 comprises a cover or cushion (not shown) arranged between the patient 15 and the field generation unit 12. The cover or cushion may be configured to protect the patient 15 from electric currents and/or enhance comfort during an imaging examination.

The field generation unit 12 may comprise or consist of one or more permanent magnets (e. g. ferromagnets), pole elements (e. g. induced magnets such as iron or other ferromagnetic materials) and/or magnet coils (e. g. based on resistive wires and/or superconducting wires). The field generation unit 12 may further comprise an array of magnets of uniform or varying magnetic field strength and magnetic field orientation. According to an embodiment, the field generation unit 12 comprises one or more planar coils and/or coils having a predefined curvature in a winding plane.

Of course, the magnetic resonance device 10 may comprise further components and/or functions which are common in magnetic resonance devices. The general operation of a magnetic resonance device 10 is known to those skilled in the art, so a more detailed description is omitted.

Fig. 2 shows an embodiment of an inventive magnetic resonance device 10 comprising a positioning unit 13 configured to adjust a position and/or an orientation of the field generation unit 12 with respect to the patient 15. For example, the positioning unit 13 may comprise a swivel joint configured to rotate the field generation unit 12 about the X-axis and/or the Y-axis. The positioning unit 13 may comprise a telescope system and/or rail mechanically connected to the support structure 11 and configured to adjust a position the field generation unit 12 along the Y-direction and/or a Z-direction. In a further example, the positioning unit 13 is configured to adjust a position of the field generation unit 12 along an X-direction and/or rotate the field generation unit 12 about the Z-direction.

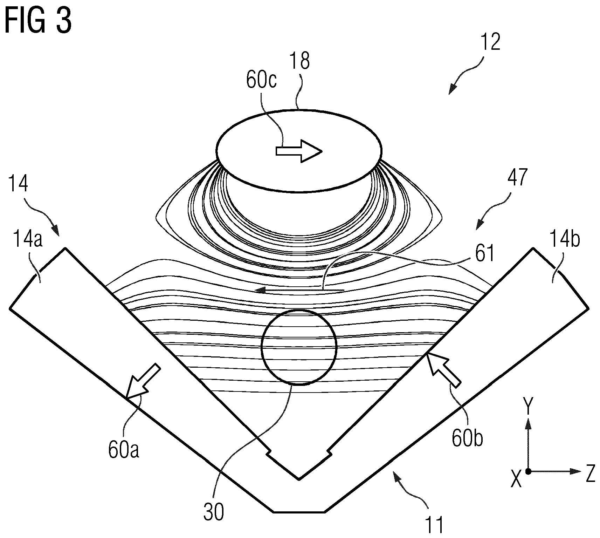

Fig. 3 shows a schematic representation of an embodiment of an inventive magnetic resonance device 10. In the depicted example, a direction of a horizontal polarization of the magnet 18 is oriented opposite to a mean or average horizontal polarization of the magnet 14. For example, polarization directions of the magnets 14a and 14b are aligned perpendicular to the surfaces of 14a and 14b facing the magnet 18, but in opposite directions (as shown by arrows 60a and 60b). However, the polarization directions may deviate from a perpendicular orientation to these surfaces. The vector sum of the two polarization directions, or the mean or average horizontal polarization of the magnet 14, will preferably be in the opposite direction to the polarization of magnet 18.

As indicated by arrows 60a, 60b, and 60c, a main direction of magnetic field lines of a magnetic field generated by the magnet 14 within the triangular half-open space 47 substantially corresponds to a main direction of magnetic field lines of a magnetic field generated by the magnet 18 within the triangular half-open space 47.

In Fig. 3, the magnet 18 is implemented as a single-sided or directional magnet configured to generate a magnetic field at a side of the magnet 18 facing towards the first magnet 14. The magnet 18 exhibits an opposite horizontal polarization with respect to the (mean or average) horizontal polarization of the magnet 14. Thus, the main direction 61 of the magnetic field lines of the magnetic fields provided by the magnets 14 and 18 within the imaging volume 30 approximately correspond to one another.

Fig. 4 depicts a schematic representation of an embodiment of an inventive magnetic resonance device 10 comprising a support structure 11 including a first section 11a attached to the magnet 14, and a second section 11b attached to the magnet 18. The first section 11a and the second section 11b can be mechanically connected or mechanically coupled via a third section 11c. However, the third section 11c may also form a part of the first section 11a and/or the second section 11b.

According to an embodiment, the support structure 11 is configured to maintain a relative spatial arrangement of the magnet 14 and the magnet 18. For example, the support structure 11 may be configured to prevent a relative movement between the magnet 14 and the magnet 18. Particularly, an adjustment unit 50 as shown in Fig. 4 may be omitted. For example, the support structure 11 may represent a static structure configured to maintain the magnet 18 in a predetermined relative position to the magnet 14.

In a preferred embodiment, the support structure 11 comprises an adjustment unit 50 (see Figs. 4 to 7) configured to variably adjust a position of the magnet 18 relative to the magnet 14. Particularly, the adjustment unit 50 may be configured to variably change a relative position between the first section 11a and the second section 11b. Referring to the example depicted in Fig. 4, the adjustment unit 50 may be configured to variably move the second section 11b along the Y-direction to increase or decrease a distance between the magnet 14 and the magnet 18.

In the embodiment shown in Fig. 4, the magnet 18 is arranged in a measurement position. The gap between the magnet 14 and the magnet 18 may be sufficient to enable a patient to access the triangular half-open space 47 comprising the imaging volume 30. In one example, the patient may access the triangular half-open space 47 along the X-direction. In a preferred embodiment, the adjustment unit 50 is configured to move the magnet 18 away from the magnet 14 and arrange the magnet 18 in an opening position, thus facilitating access to the triangular half-open space 47.

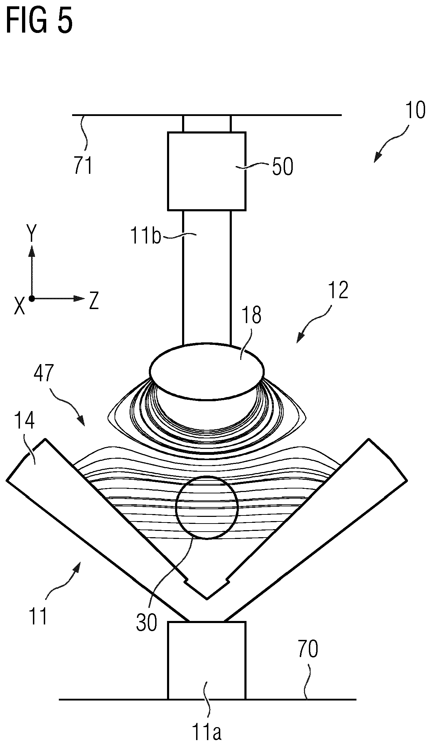

Fig. 5 depicts a further embodiment of an inventive magnetic resonance device 10. In the depicted example, the support structure 11 comprises a first section 11a attached to the magnet 14, and a second section 11b attached to the magnet 18. The first section 11a and the second section 11b are mechanically separated or disjoint.

For example, the first section 11a may be mechanically connected to a floor 70 of an examination room, whereas the second section 11b may be mechanically connected to a wall and/or a ceiling 71 of the examination room.

The support structure 11 may be a static structure configured to permanently arrange the magnet 18 in a measurement position. In a preferred embodiment, however, the support structure 11 comprises an adjustment unit 50 configured to variably change a relative position between the first section 11a and the second section 11b. Particularly, the adjusting unit 50 may be configured to move the magnet 18 towards the magnet 14 or away from the magnet 14 along the Y-direction.

Fig. 6 shows an embodiment of an inventive magnetic resonance device 10 which is configured as a chair. In the depicted example, the magnetic resonance device 10 is mounted to a floor 70 of an examination room via the support structure 11. The first section 11a of the support structure 11 provides mechanical support to the magnet segments 14a and 14b forming the triangular half-open space 47.

The support structure 11 comprises an adjustment unit 50, which is configured as a pivot or swivel. The adjustment unit 50 comprises a compression spring or air ram 50a, which is configured to rotate the second section 11b carrying the magnet 18 about the pivot 50. The control unit 20 of magnetic resonance device 10 may be configured to control the air ram 50a to move the magnet 18 between a measurement position and an opening position in an automated manner. In the depicted example, the magnet 18 is arranged in the opening position to allow for a patient to access the triangular half-open space 47, particularly to allow for the patient to take a seat on the magnet 14. The arrow 60 indicates a motion trajectory of the magnet 18 moving from the opening position to the measurement position and vice versa.

The magnetic resonance device 10 may comprise a stop element 51 configured to restrict or limit a movement of the magnet 18 along the motion trajectory 60. In the embodiment depicted in Fig. 6, the magnetic resonance device 10 comprises a lock 51b configured to mechanically engage with a tubular section 51a of the second section 11b. The lock 51b is attached to the support structure 11 via dedicated support elements which are configured to prevent the magnet 18 from moving past the measurement position and potentially hurt a patient 15 accommodated within the triangular half-open space 47.

In a preferred embodiment, the field generation unit 12 is configured in such a way that a spatial location of an imaging volume 30 provided via the magnet 14 and the magnet 18 corresponds to a spatial location of a prostate of a patient 15 sitting on the magnet 14 in an intended position when the magnet 18 is arranged in the measurement position.

In the example depicted in Fig. 6, the magnetic resonance device 10 comprises a gradient field system 27 including at least one gradient coil 28a arranged adjacent to the magnet 14 and at least one gradient coil 28b arranged adjacent to the magnet 18. Furthermore, the magnetic resonance device comprises a radiofrequency system 29 including at least one radiofrequency antenna 32a arranged adjacent to the gradient coil 28a and at least one radiofrequency coil 32b arranged adjacent to the gradient coil 28b. In distributing the gradient coils 28 of the gradient field system 27 and the radiofrequency antennas 32 of the radiofrequency system 29 over the magnet 14 and the magnet 18, a coverage of an object positioned within the imaging volume 30 may be increased. Thus, a quality of magnetic resonance image data acquired via the magnetic resonance device 10 may favourably be improved. In the depicted embodiment, the gradient system 27 comprises a gradient coil wire 27a configured to electrically connect the gradient coils 28a and 28b to the gradient control unit 21 (see Fig. 1).

According to an embodiment, parts of the field generation unit 12 and/or the support structure 11 are upholstered or lined with cushions to improve comfort of a patient 15 accommodated within the triangular half-open space 47.

Fig. 7 shows a further embodiment of an inventive magnetic resonance device 10 configured as a chair or couch. In accordance with the embodiment depicted in Fig. 6, the magnetic resonance device 10 is mounted to a floor 70 of an examination room via the support structure 11. The first section 11a of the support structure 11 provides mechanical support to the magnet segments 14a and 14b forming a triangular half-open space 47.

In the depicted embodiment, the support structure 11 comprises a third section 11c mechanically coupled to an adjusting unit 50. The adjustment unit 50 is configured to move the magnet 18 between the measurement position and the opening position along a substantially linear motion trajectory represented by arrow 60.

The adjusting unit 50 may comprise rods 50b acting as guide elements and bearings 50a which allow the magnet 18 and the rods 50b to move along the X-direction.

The support element 11 may further comprise a bracing element 11d confining the magnet 14 in the X-direction. The bracing element 11d may provide a stop element configured to prevent the magnet 18 from moving beyond the measurement position. Preferably, the bracing element 11d comprises receptacles 11e configured to accommodate end sections of the guide elements 50b when the magnet 18 is moved to the measurement position.

In an alternative embodiment, the guide elements or rods 50b may be permanently accommodated within the receptacles 11e and the second section 11b carrying the magnet 18 may be configured to slide along the rods 50b in the X-direction. For example, the second section 11b may comprises bearings allowing the second section 11b and the magnet 18 to slide along the guide elements 50b.

In accordance with the embodiment depicted in Fig. 6, the gradient field system 27 comprises at least one gradient coil 28a arranged adjacent to the magnet 14 and at least one gradient coil 28b arranged adjacent to the magnet 18. The gradient coils 28 of the gradient system 27 may be arranged on a surface of the magnet 14 and/or the magnet 18 facing towards the imaging volume 30. It is also conceivable that the gradient coils 28 are accommodated within a recess in the surface facing towards the imaging volume 30 of the magnet 14 and/or the magnet 18. The gradient system 27 may comprise a gradient coil wire 27a electrically connecting the gradient coil 28a and the gradient coil 28b to the gradient control unit 21 (see Fig. 1).

In the embodiment shown in Fig. 7, the magnetic resonance device 10 comprises a radiofrequency system 29 including at least one radiofrequency antenna 32a arranged adjacent to the gradient coil 28a and at least one radiofrequency coil 32b arranged adjacent to the gradient coil 28b.

In certain embodiments, one or more gradient coil 28 may be arranged adjacent to the magnet 14 and/or the magnet 18. Likewise, one or more radiofrequency coils 32 may be arranged adjacent to the magnet 14 and/or the magnet 18.

In a preferred embodiment, the supporting structure 11 of the magnetic resonance device 10 comprises a yoke attached to the magnet 14. The yoke may be configured to modify and/or shape a magnetic field generated by the field generation unit 12. It is conceivable that the yoke is positioned on a side of the field generation unit 12 facing away from the triangular half-open space 47, thus ensuring best possible access to the imaging volume 30. The yoke may correspond to the first section 11a of the support structure 11 as shown in Figs. 6 and 7. However, the yoke may also be mechanically connected to the magnet 18 of the field generation 12.

According to an embodiment, the support structure 11 or parts of the support structure 11 are movable and can be opened and/or tilted to facilitate an access to the imaging volume 30. For example, the support structure 11 may comprise a hinge or other form of movable joint configured to modify the angle and/or the relative position between the end plates 45a, 45b and/or the magnet segments 14a, 14b. Such an adjustment may facilitate an access to the triangular half-open space 47 and/or allow for a shape and/or a spatial position of the imaging volume 30 provided by the field generation unit 12 to be modified to match a location of a specific imaging object.

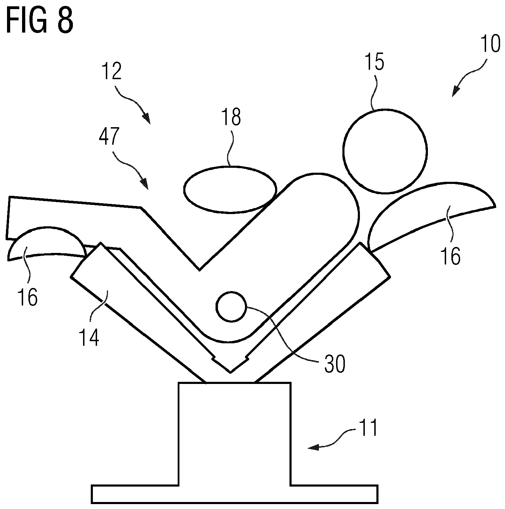

Fig. 8 shows a preferred embodiment of an inventive magnetic resonance device 10. In the depicted example, the magnetic resonance device 10 is configured as a dedicated scanner for imaging the prostate of a patient 15. In accordance with the embodiments depicted in Figs. 6 and 7, the field generation unit 12 is formed like a chair allowing the patient 15 to be accommodated within the triangular half-open space 47 in a sitting position. Particularly, the field generation unit 12 may be configured in such a way that a spatial position of the imaging volume 30 corresponds to a spatial position of the prostate of the patient 15 accommodated within the triangular half-open space 47.

The magnetic resonance device 10 may comprise a dedicated patient support 16 configured to support one or more body regions of the patient 15, such as a head or a foot, during a magnetic resonance measurement.

The embodiments described herein are to be recognized as examples. It is to be understood that individual embodiments may be extended by or combined with features of other embodiments if not stated otherwise. The embodiments depicted in the Figs. 1 to 8 are representations that do not have to be to scale.

## Claims

1. A magnetic resonance device (10) comprising a field generation unit (12) configured to generate a main magnetic field including an imaging volume (30), and a support structure (11) configured to structurally support the field generation unit (12),
wherein the field generation unit (12) comprises a first magnet (14) and a second magnet (18), wherein the first magnet (14) comprises two magnet segments (14a; 14b) arranged at an angle to each other to form a triangular half-open space (47) which encloses at least a part of the imaging volume (30),
and wherein the first magnet (14) and the second magnet (18) are arranged at opposing sides of the imaging volume (18).

2. The magnetic resonance device (10) according to claim 1, wherein a direction of a horizontal polarization of the second magnet (18) is opposite to a horizontal polarization of the first magnet (14).

3. The magnetic resonance device (10) according to claim 1 or claim 2, wherein the support structure (11) is configured to variably change a spatial position of the second magnet (18) relative to the first magnet (14).

4. The magnetic resonance device (10) according to claim 3, wherein the support structure (11) is configured to arrange the second magnet (18) in an opening position and a measurement position.

5. The magnetic resonance device (10) according to claim 4, wherein the field generation unit (12) is configured in such a way to cause the imaging volume (30) to move away from a corner (41) of the triangular half-open space (47) when the second magnet (18) is moved from the opening position to the measurement position.

6. The magnetic resonance device (10) according to one of the claims 3 to 5, wherein the field generation unit (12) is configured in such a way to cause a spatial position of the imaging volume (30) to change when the spatial position of the second magnet (18) is changed relative to the first magnet (14).

7. The magnetic resonance device (10) according to one of the claims 3 to 6, wherein the support structure (11) comprises a first section (11a) attached to the first magnet (14), and a second section (11b) attached to the second magnet (18), wherein the first section (11a) is mechanically connected to the second section (11b) or wherein the first section (11a) and the second section (11b) are mechanically disjoint.

8. The magnetic resonance device (10) according to claim 7, wherein the first section (11a) is mechanically coupled to the second section (11b) and wherein the support structure (11) comprises an adjustment unit (50) configured to variably change a relative position between the first section (11a) and the second section (11b).

9. The magnetic resonance device (10) according to claim 8, wherein the adjustment unit (50) comprises a moveable joint.

10. The magnetic resonance device (10) according to one of the claims 8 or 9, wherein the adjustment unit (50) comprises a guide mechanism.

11. The magnetic resonance device (10) according to one of the claims 8 to 10, comprising a stop element (51) configured to limit a movement of the second magnet (18) along a motion trajectory (60) defined by the adjustment unit (50).

12. The magnetic resonance device (10) according to one of the claims 3 to 11, wherein the support structure (11) is configured to arrange the second magnet (18) in a first measurement position and a second measurement position, wherein a spatial location of the second magnet (18) in the first measurement position differs from a spatial position of the second magnet (18) in the second measurement position.

13. The magnetic resonance device (10) according to one of the preceding claims, comprising a radiofrequency system (29) including at least one radiofrequency antenna (32) configured to transmit and/or receive radiofrequency radiation.

14. The magnetic resonance device (10) according to one of the preceding claims, comprising a gradient field system (27) including at least one gradient coil (28) configured to generate at least one magnetic gradient field.
